# EUROPÄISCHE PATENTSCHRIFT

(11) **EP 3 122 271 B1**
(45) Veröffentlichungstag und Bekanntmachung des Hinweises auf die Patenterteilung: **22.01.2020**
(21) Anmeldenummer: 15718345.0
(22) Anmeldetag: 17.03.2015
(51) Int. Cl.: A61B 17/72, A61B 17/00

(54) **VERFAHREN ZUM HERSTELLEN EINES IMPLANTATS FÜR DIE OSTEOSYNTHESE UND KNOCHENNAGEL**
METHOD OF FABRICATING AN OSTEOSYNTHESIS IMPLANT AND BONE NAIL
PROCÉDÉ DE FABRICATION D'UN IMPLANT POUR L'OSTÉOSYNTHÈSE ET CLOU ORTHOPÉDIQUE

(30) Priorität: 24.03.2014 DE 102014103972
(43) Veröffentlichungstag der Anmeldung: 01.02.2017
(73) Patentinhaber: GLW, Inc., Edgewater, NJ 07020 (US)
(72) Erfinder: LUTZ Christian, 24226 Heikendorf (DE)
(74) Vertreter: Cole, Douglas Lloyd
(86) Internationale Anmeldenummer: PCT/DE2015/100109
(87) Internationale Veröffentlichungsnummer: WO 2015/144131

(56) Entgegenhaltungen:
- WO-A1-2007/101267
- WO-A1-2008/134264
- WO-A1-2011/082152
- WO-A1-2012/065068
- WO-A1-2014/015262
- US-A1- 2006 247 638
- US-A1- 2011 208 189

## Beschreibung

Die Erfindung betrifft ein Verfahren zum Herstellen eines Implantats für die Osteosynthese. Die Erfindung betrifft auch einen Knochennagel.

In der Traumatologie verwendete Implantate für die Osteosynthese sind beispielsweise aus der EP 1 488 752 A1 bekannt.

Nachteilig an diesem Produkt sind die zur Herstellung der körpergerechten Form als auch der lasttragende Funktion zum Teil sehr arbeits- und zeitaufwändigen spanenden Fertigungsverfahren. Diese Verfahren benötigen entsprechende Zeiten in der Fertigung und komplexe und zeitintensive Verfahren in der Qualitätskontrolle. Wenngleich diese Verfahren in den letzten zehn Jahren weitgehend optimiert wurden, bieten diese heute nur noch sehr wenig Potential zur Reduzierung des Arbeits- und damit auch Kostenaufwands.

Implantate für die Osteosynthese sind des Weiteren auch aus der WO 2014/015262 und der WO 2008/134264 bekannt.

Aufgabe der Erfindung ist es daher, ein Verfahren zum Herstellen eines Implantats für die Osteosynthese zu schaffen, das weitgehend ohne zeit- und arbeitsaufwändige spanende Herstellungstechniken auskommt und zu einem kostengünstig herzustellenden und zugleich qualitativ hochwertigem Produkt führt.

Diese Aufgabe wird durch den Knochennagel mit den Merkmalen von Anspruch 1 und dem Verfahren mit den Merkmalen von Ansprüchen 12, 15 und 16 gelöst. Die Unteransprüche geben jeweils vorteilhafte Ausgestaltungen der Erfindung wieder.

Grundgedanke der Erfindung ist es, den lasttragenden Teil eines Implantats durch ein Massenherstellungsverfahren, wie z.B. Strangpressen, Stanzen, Schmieden oder Gießen eines geeigneten Profils oder Rohlings, zu erzeugen, wobei die körpergerechte bzw. körperangepasste Form des Implantats durch Umspritzen des Profils/Rohlings mit einem implantierbaren Kunststoff erfolgen soll. Die nachteiligen Eigenschaften des einen Verfahrens werden dabei durch die Vorteile des jeweils anderen Verfahrens aufgehoben: ein im Spritzgussverfahren oder beim Fließpressen verwendbarer Kunststoff allein besäße nicht die ausreichende Festigkeit und würde brechen, wohingegen ein günstig hergestelltes Profil keine körpergerechte Form aufweisen und für den Patienten unverträglich sein würde. Beide Verfahren zusammen aber sind verhältnismäßig einfach kostengünstig in der Anwendung und erfüllen in der Kombination die Aufgaben der Lastaufnahme und der Körperverträglichkeit. Das erfindungsgemäße Verfahren zum Herstellen eines Implantats für die Osteosynthese besteht damit im Wesentlichen aus den Schritten Anfertigen eines lasttragenden Bauelements aus Metall und Ummanteln des lasttragenden Bauelements mit einem biokompatiblen Material.

Das Ummanteln erfolgt bevorzugt durch Umspritzen. Alternativ kann das Ummanteln auch durch Fließpressen erfolgen.

Alternativ zu einem vollständigen Umspritzen des lasttragenden Bauelements kann das lasttragende Bauelement auch nicht vollständig, sondern nur abschnittsweise ummantelt sein. So ist es möglich, ein Implantat für die Osteosynthese zu schaffen, dass aus einem metallischen Abschnitt und einen aus einem Kunststoff gefertigten Abschnitt besteht. Der metallische Abschnitt muss weiterhin aus einem hochwertigen Metall gearbeitet sein, wobei die Anfertigung eines aus Kunststoff bestehenden Abschnitts allerdings weiterhin Kosten zu sparen vermag.

Bevorzugt erfolgt das Anfertigen des lasttragenden Bauelements weitestgehend mittels Strangpressen oder Stanzen, wobei das verwendete Metall in einem Vergleichsbeispiel Stahl ist. Stahl hat den Vorteil, dass er weniger kerbempfindlich als Titan und in der Beschaffung günstiger ist. Durch die höhere Steifigkeit und die bessere Umformbarkeit von Stahl ist es ermöglicht, ein Profil zu erzeugen, das die gleiche Steifigkeit wie ein Titanimplantat erreicht, aber mit weniger Material auskommt.

Das lasttragende Bauelement kann aber auch - sofern dieses mit geringem Aufwand erledigt werden kann - mittels spanender Fertigungsverfahren hergestellt werden.

Das verwendete Metall kann aber auch eine Cobalt-Molybdän-Legierung, insbesondere eine Chrom-Cobalt-Molybdän-Legierung sein. Erfindungsgemäss ist das verwendete Metall Titan.

Das biokompatible Material hingegen ist bevorzugt ein Polymer. Besonders bevorzugt ist das Polymer ausgewählt aus der Gruppe von Polymeren bestehend aus Polyetheretherketon (PEEK), mit Kohlefaser verstärktem Polyetheretherketon und Polyamid (PA).

Alternativ kann als biokompatibles Material auch Titan verwendet werden
In jedem Fall wird die äußere Form des Implantats durch das das lasttragende Bauelement ummantelte biokompatible Material bestimmt, sodass das Umspritzen oder Fließpressen im Wesentlichen auch die Formgebung des Implantats bestimmt. Es kann also der Fall vorliegen, dass die äußere Form des Implantats ausschließlich durch die äußere Form des biokompatiblen Materials gebildet ist, d.h. sich die äußere Form des lasttragenden Bauelements und die äußere Form des biokompatiblen Materials zueinander unähnlich, also unterschiedlich sind.

Nach einer weiteren besonders bevorzugten Ausgestaltung der Erfindung wird das biokompatible Material weiter als Medikamententräger für Medikamente insbesondere zur Verhinderung von Infektionen oder zur Beschleunigung des Knochenwachstums ausgebildet. Das Implantat ist bevorzugt ein Knochen- oder Marknagel oder eine Knochenplatte.

Speziell handelt es sich um einen Knochennagel für die Osteosynthese, mit einem Metall-Inlay, das als lasttragendes Bauelement ausgebildet ist, und einem durch Ummanteln, insbesondere durch Umspritzen oder durch Fließpressen wenigstens eines Teilbereichs des Metall-Inlays mit dem Metall-Inlay verbundenen Kunststoffkörper, der im Wesentlichen die äußere Form des Knochennagels bestimmt. Der Knochennagel ist insbesondere durch das erfindungsgemäße Verfahren hergestellt.

Erfindungsgemäss weist der Knochennagel eine in dem ummantelten Teilbereich des Metall-Inlays quer zu dessen Längsachse eingebrachte Nut auf. Diese Nut, in die der Kunststoffkörper eingreift, dient zur Fixierung des Kunststoffkörpers am Metall-Inlay. Die Nut ist insbesondere das Metall-Inlay umlaufend ausgebildet, sodass der Kunststoffkörper gegen ein axiales Verrutschen am Metall-Inlay gesichert ist.

Nach einer weiteren bevorzugten Ausgestaltung weist der Knochennagel einen sich axial vom Metall-Inlay erstreckenden, ein Abstützen des Kunststoffkörpers am Metall-Inlay bewirkenden Anschlag auf. Der Umfang des Anschlag kann insbesondere auch dazu verwendet werden, dass Metall-Inlay beim Umspritzen oder Fließpressen besser greifen zu können, sodass das Metall-Inlay am Anschlag in die entsprechende Vorrichtung eingespannt werden kann - der Außenumfang des Anschlags wird dabei nicht ummantelt.

Speziell ist auch vorgesehen, dass der ummantelte Teilbereich des Metall-Inlays wenigstens abschnittsweise konisch ausgebildet ist. Durch diese Ausbildung wird für eine möglichst gleichmäßige Kraftüberleitung vom Metalleinsatz in den Kunststoffkörper gesorgt.

Nach einem anderen Aspekt der Erfindung weist der ummantelte Abschnitt des Metall-Inlays ein Gewinde auf, wobei der Gewindegrund und die Gewindespitzen insbesondere abgerundet sind, um eine den Kunststoffkörper beeinträchtigende Kerbwirkung zu vermeiden. Speziell kann das Gewinde von wenigstens einer axial verlaufenden Nut durchbrochen sein, wobei besonders bevorzugt drei derartige Nuten in einem Winkel von je 120° zueinander vorgesehen sind. Diese Ausbildung ermöglicht eine besonders stabile Verbindung von Metall-Inlay und Kunststoffkörper zueinander.

Schließlich kann der Knochennagel in dem distalen Bereich des Kunststoffkörpers ein ummanteltes zweites Metall-Inlay mit einer Öffnung zur Aufnahme einer Verriegelungsschraube aufweisen.

Die Erfindung wird anhand einiger Ausführungsbeispiele nach der Erfindung näher erläutert. Es zeigen:
- Fig. 1: einen Querschnitt eines Knochennagels nach einem ersten Ausführungsbeispiel;
- Fig. 2: einen Querschnitt eines Knochennagels nach einem zweiten Ausführungsbeispiel;
- Fig. 3: einen Querschnitt eines Knochennagels nach einem dritten Ausführungsbeispiel; und
- Fig. 4: einen Querschnitt eines Knochennagels nach einem vierten Ausführungsbeispiel.

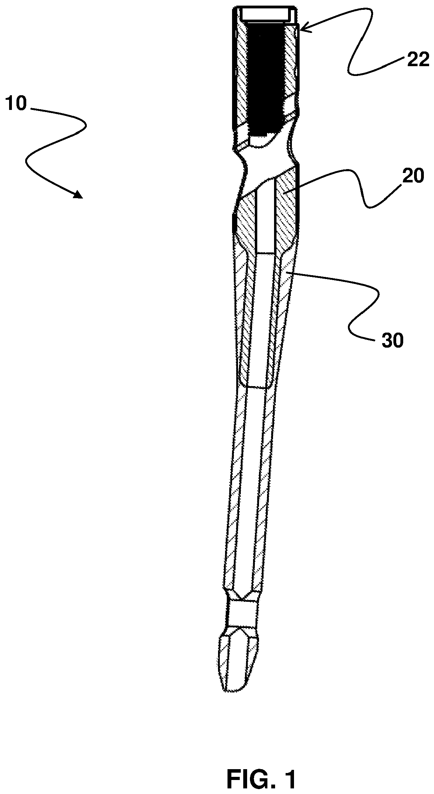

Fig. 1 zeigt einen Querschnitt eines Knochennagels (oder Marknagels) nach einem ersten Ausführungsbeispiel. Der Knochennagel 10 entspricht in seiner äußeren Formgebung im Wesentlichen der Form von herkömmlichen Knochen- oder Marknägeln.

Der Knochennagel 10 besteht erfindungsgemäß aus einem als lasttragendes Bauelement ausgebildeten Metall-Inlay 20 und einem mit diesem verbundenen Kunststoffkörper 30 aus einem biokompatiblen Polymer.

Bei diesem ersten Ausführungsbeispiel ist speziell vorgesehen, dass die gesamte Oberfläche - mit Ausnahme der proximalen Stirnseite des Knochennagels 10 - vollständig mit dem biokompatiblen Material umspritzt ist.

Das Metall-Inlay 20 weist in seinem proximalen Bereich zwei das Metall-Inlay 20 umlaufende Nuten 22 auf, in die der Kunststoffkörper 30 eingreift und dadurch gegen ein Verrutschen gegenüber dem Metall-Inlay 20 gesichert ist.

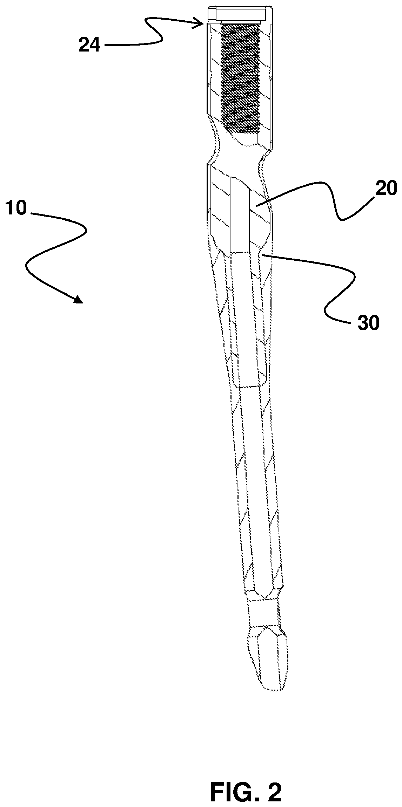

Fig. 2 zeigt einen Querschnitt eines Knochennagels nach einem zweiten Ausführungsbeispiel. Der in Fig. 2 gezeigte Knochennagel 10 entspricht im Wesentlichen dem in Fig. 1 gezeigten Ausführungsbeispiel mit dem Unterschied, dass zusätzlich ein die axiale Erstreckung des Kunststoffkörpers 30 begrenzender Anschlag 24 vorgesehen ist, der sich radial vom Metall-Inlet 20 erstreckt.

Die Mantelfläche des Anschlags 24 und die Oberfläche des Kunststoffkörpers 30 sind fluchtend ausgebildet, sodass der Anschlag 24 des Metall-Inlays 20 und der Kunststoffkörper 30 dichtend aneinander liegen.

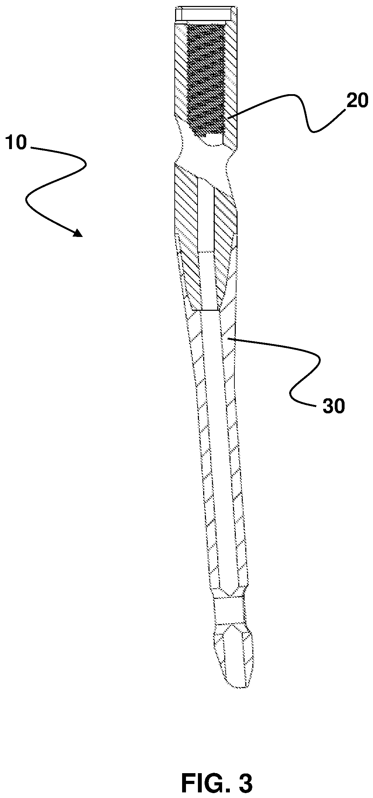

Fig. 3 zeigt nun einen Querschnitt eines Knochennagels nach einem dritten Ausführungsbeispiel. Das Metall-Inlay 20 ist in seinem distalen Abschnitt konisch ausgebildet, sodass hiermit eine möglichst gleichmäßige Kraftüberleitung vom Metalleinsatz 20 in den Kunststoffkörper 30 erreicht wird.

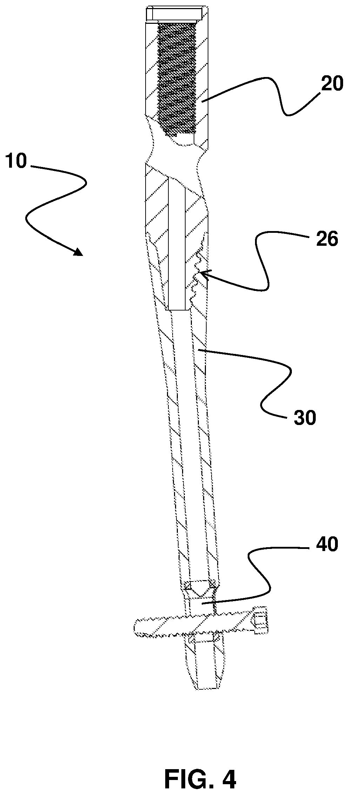

Schließlich zeigt Fig. 4 einen Knochennagel nach einem vierten Ausführungsbeispiel. Der Knochennagel 10 gemäß viertem Ausführungsbeispiel zeigt die Besonderheit eines Metall-Inlays 20 dessen distaler konischer Abschnitt zur besseren Verbindung mit dem Kunststoffkörper 30 mit einem Gewinde 26 versehen ist.

Zusätzlich weist dieser Knochennagel 10 die Besonderheit eines im distalen Bereich des Kunststoffkörpers 30 umspritztes zweites Metall-Inlay 40 auf, das von einer Verriegelungsschraube eingenommen ist. Dieser weitere Metalleinsatz bietet ein gutes Interface für die distale metallische Verriegelungsschraube.

## Patentansprüche

1. Knochennagel (10) für die Osteosynthese, mit
- einem lasttragenden Bauelement (20) aus Metall, wobei das lasttragende Bauelement ein proximales Ende, ein distales Ende, einen konischen Abschnitt zwischen dem proximalen Ende und dem distalen Ende, und eine Längsachse aufweist, und
- einem durch Ummanteln des lasttragenden Bauelements mit einem biokompatiblen Material (30) mit dem lasttragenden Bauelement verbundenen Körper,
**dadurch gekennzeichnet, dass** das lasttragende Bauelement (20) in seinem distalen Abschnitt konisch ausgebildet ist, wobei
das biokompatible Material über dem konischen Abschnitt angeordnet ist;
wobei das Metall Titan ist,
und das lasttragende Bauelement (20) eine quer zu dessen Längsachse eingebrachte Nut (22) aufweist in die das biokompatible Material eingreift.

2. Knochennagel (10) nach Anspruch 1, **dadurch gekennzeichnet, dass** die Nut das lasttragende Bauelement umlaufend ausgebildet ist.

3. Knochennagel (10) nach einem der vorhergehenden Ansprüche, **gekennzeichnet durch** einen am proximalen Ende des lasttragenden Bauelements vorgesehenen, sich radial vom lasttragenden Bauelement (20) erstreckenden, und die axiale Erstreckung des biokompatiblen Materials (30) begrenzenden Anschlag (24).

4. Knochennagel (10) nach Anspruch 3, **dadurch gekennzeichnet, dass** eine Mantelfläche des Anschlags und eine Oberfläche des biokompatiblen Materials fluchtend ausgebildet sind, sodass der Anschlag und das biokompatible Material dichtend aneinander liegen.

5. Knochennagel (10) nach einem der vorhergehenden Ansprüche, **dadurch gekennzeichnet, dass** der konische Abschnitt ein Gewinde (26) aufweist, und dass das biokompatible Material über dem Gewinde angeordnet ist.

6. Knochennagel (10) nach Anspruch 5, **dadurch gekennzeichnet, dass** das Gewinde abgerundete Gewindespitzen und einen abgerundeten Gewindegrund aufweist.

7. Knochennagel (10) nach einem der Ansprüche 5 und 6, **dadurch gekennzeichnet, dass** das Gewinde (26) von wenigstens einer axial verlaufenden Nut durchbrochen ist.

8. Knochennagel (10) nach Anspruch 7, **dadurch gekennzeichnet, dass** drei derartige, in einem Winkel von je 120° zueinander angeordnete Nuten vorgesehen sind, wobei das Gewinde von jeder der drei Nuten durchbrochen wird.

9. Knochennagel (10) nach einem der vorhergehenden Ansprüche, **gekennzeichnet durch** ein im distalen Bereich des Kunststoffkörpers (30) ummanteltes Metall-Inlay (40) mit einer Öffnung zur Aufnahme einer Verriegelungsschraube.

10. Knochennagel (10) nach einem der vorhergehenden Ansprüche, **dadurch gekennzeichnet, dass** das biokompatible Material ein Polymer ist.

11. Knochennagel (10) nach Anspruch 10, **dadurch gekennzeichnet, dass** das Polymer ausgewählt ist aus der Gruppe von Polymeren bestehend aus Polyetheretherketon (PEEK), mit Kohlefaser verstärktem Polyetheretherketon und Polyamid (PA).

12. Verfahren zum Herstellen eines Implantats (10) für die Osteosynthese, mit den Schritten:
- Anfertigen eines lasttragenden Bauelements (20) aus Metall, sodass das lasttragende Bauelement ein proximales Ende, ein distales Ende, einen konischen Abschnitt zwischen dem proximalen Ende und dem distalen Ende, und eine Längsachse aufweist, und
- Ummanteln des lasttragenden Bauelements mit einem biokompatiblen Material (30), sodass das biokompatible Material über dem konischen Abschnitt angeordnet ist;
wobei das Metall Titan ist,
und das lasttragende Bauelement (20) eine quer zu dessen Längsachse eingebrachte Nut (22) aufweist in die das biokompatible Material eingreift.

13. Verfahren nach Anspruch 12, **dadurch gekennzeichnet, dass** das Ummanteln mittels Umspritzen erfolgt.

14. Verfahren nach einem der Ansprüche 12 und 13, **dadurch gekennzeichnet dass** das Implantat ein Knochennagel ist.

15. Verfahren zum Herstellen eines Implantats für die Osteosynthese, mit den Schritten:
Anfertigen eines lasttragenden Bauelements (20) aus Titan, sodass das lasttragende Bauelement folgendes aufweist:
ein proximales Ende mit einem Anschlag (24), der sich radial vom lasttragenden Bauelement erstreckt und eine Mantelfläche aufweist;
ein distales Ende;
einen zwischen dem proximalen Ende und dem distalen Ende angeordneten konischen Abschnitt mit einem Gewinde mit abgerundeten Gewindespitzen und abgerundetem Gewindegrund, wobei
dass Gewinde (26) von wenigstens einer axial verlaufenden Nut durchbrochen ist, und eine Längsachse; und
Ummanteln eines Abschnitts des lasttragenden Bauelements mit einem biokompatiblen Material (30), so dass das biokompatible Material über dem Gewinde angeordnet ist, wobei die Mantelfläche des Anschlags und eine Oberfläche des biokompatiblen Materials fluchtend ausgebildet sind, sodass der Anschlag und das biokompatible Material dichtend aneinander liegen.

16. Verfahren zum Herstellen eines Implantats für die Osteosynthese, mit den Schritten:
Anfertigen eines lasttragenden Bauelements (20) aus Titan, sodass das lasttragende Bauelement folgendes aufweist:
ein proximales Ende mit einem Anschlag (24), der sich radial vom lasttragenden Bauelement erstreckt und eine Mantelfläche aufweist;
ein distales Ende;
einen zwischen dem proximalen Ende und dem distalen Ende angeordneten konischen Abschnitt mit einem Gewinde mit abgerundeten Gewindespitzen und abgerundetem Gewindegrund, wobei
dass Gewinde (26) von drei axial verlaufenden Nuten durchbrochen ist, und eine Längsachse; und
Umspritzen eines Abschnitts des lasttragenden Bauelements mit einem biokompatiblen Polymer (30), sodass das biokompatible Polymer über dem Gewinde und den drei axial verlaufenden Nuten angeordnet ist, wobei die Mantelfläche des Anschlags und eine Oberfläche des biokompatiblen Polymers fluchtend ausgebildet sind, sodass der Anschlag und das biokompatible Material dichtend aneinander liegen.

## Claims

1. Bone nail (10) for osteosynthesis, comprising
- a load-bearing component (20) made of metal, the load-bearing component comprising a proximal end, a distal end, a conical portion between the proximal end and the distal end, and a longitudinal axis, and
- a body connected to the load-bearing component by means of the load-bearing component being sheathed in a biocompatible material (30),
**characterised in that** the distal portion of the load-bearing component (20) is designed to be conical,
the biocompatible material being arranged over the conical portion;
the metal being titanium
and the load-bearing component (20) comprising a groove (22) made transversely to the longitudinal axis thereof, in which groove the biocompatible material engages.

2. Bone nail (10) according to claim 1,
**characterised in that** the groove is designed to extend around the load-bearing component.

3. Bone nail (10) according to either of the preceding claims, **characterised by** and end stop (24) provided on the proximal end of the load-bearing component, extending radially from the load-bearing component (20), and delimiting the axial extension of the biocompatible material (30).

4. Bone nail (10) according to claim 3, **characterised in that** a lateral surface of the end stop and a surface of the biocompatible material are designed to be aligned such that the end stop and the biocompatible material adjoin one another tightly.

5. Bone nail (10) according to any of the preceding claims, **characterised in that** the conical portion comprises a thread (26), and **in that** the biocompatible material is arranged over the thread.

6. Bone nail (10) according to claim 5, **characterised in that** the thread comprises rounded thread crests and a rounded thread root.

7. Bone nail (10) according to any of claims 5 and 6, **characterised in that** the thread (26) is crossed by at least one axially extending groove.

8. Bone nail (10) according to 7, **characterised in that** three such grooves arranged at an angle of 120° to one another are provided, the thread being crossed by each of the three grooves.

9. Bone nail (10) according to any of the preceding claims, **characterised by** a metal inlay (40) sheathed in the distal region of the plastic body (30) and comprising an opening for receiving a locking screw.

10. Bone nail (10) according to any of the preceding claims, **characterised in that** the biocompatible material is a polymer.

11. Bone nail (10) according to claim 10, **characterised in that** the polymer is selected from the group of polymers consisting of polyether ether ketone (PEEK), carbon-fibre-reinforced polyether ether ketone and polyamide (PA).

12. Method for producing an implant (10) for osteosynthesis, comprising the steps of:
- manufacturing a load-bearing component (20) from metal such that the load-bearing component comprises a proximal end, a distal end, a conical portion between the proximal end and the distal end, and a longitudinal axis, and
- sheathing the load-bearing component in a biocompatible material (30) such that the biocompatible material is arranged over the conical portion;
wherein the metal is titanium,
and the load-bearing component (20) comprises a groove (22) made transversely to the longitudinal axis thereof, in which groove the biocompatible material engages.

13. Method according to claim 12, **characterised in that** the sheathing takes place by means of overmoulding.

14. Method according to any of claims 12 and 13, **characterised in that** the implant is a bone nail.

15. Method for producing an implant for osteosynthesis, comprising the steps of:
manufacturing a load-bearing component (20) from titanium such that the load-bearing component comprises the following:
a proximal end comprising an end stop (24) which extends radially from the load-bearing component and comprises a lateral surface;
a distal end;
a conical portion arranged between the proximal end and the distal end and comprising a thread having rounded thread crests and a rounded thread root, wherein the thread (26) is crossed by at least one axially extending groove, and
a longitudinal axis; and
sheathing a portion of the load-bearing component in a biocompatible material (30) such that the biocompatible material is arranged over the thread, wherein the lateral surface of the end stop and a surface of the biocompatible material are designed to be aligned such that the end stop and the biocompatible material adjoin one another tightly.

16. Method for producing an implant for osteosynthesis, comprising the steps of:
manufacturing a load-bearing component (20) from titanium such that the load-bearing component comprises the following:
a proximal end comprising an end stop (24) which extends radially from the load-bearing component and comprises a lateral surface;
a distal end;
a conical portion arranged between the proximal end and the distal end and comprising a thread having rounded thread crests and a rounded thread root, wherein the thread (26) is crossed by three axially extending grooves,
and a longitudinal axis; and
overmoulding a portion of the load-bearing component with a biocompatible polymer (30) such that the biocompatible polymer is arranged over the thread and the three axially extending grooves, wherein the lateral surface of the end stop and a surface of the biocompatible polymer are designed to be aligned such that the end stop and the biocompatible material adjoin one another tightly.

## Revendications

1. Clou orthopédique (10) destiné à l'ostéosynthèse, comprenant
- un composant porteur (20) fabriqué en métal, le composant porteur comprenant une extrémité proximale, une extrémité distale, une partie conique entre l'extrémité proximale et l'extrémité distale, et un axe longitudinal, et
- un corps connecté au composant porteur au moyen du composant porteur étant gainé dans un matériau biocompatible (30),
**caractérisé en ce que** la partie distale du composant porteur (20) est conçue pour être conique,
le matériau biocompatible étant agencé sur la partie conique ;
le métal étant le titane
et le composant porteur (20) comprenant une rainure (22) faite de manière transversale par rapport à l'axe longitudinal de celui-ci, dans laquelle rainure le matériau biocompatible entre en prise.

2. Clou orthopédique (10) selon la revendication 1, **caractérisé en ce que** la rainure est conçue pour s'étendre autour du composant porteur.

3. Clou orthopédique (10) selon l'une ou l'autre des revendications précédentes, **caractérisé par** une butée d'extrémité (24) fournie sur l'extrémité proximale du composant porteur, s'étendant de manière radiale à partir du composant porteur (20), et délimitant l'extension axiale du matériau biocompatible (30).

4. Clou orthopédique (10) selon la revendication 3, **caractérisé en ce qu'**une surface latérale de la butée d'extrémité et une surface du matériau biocompatible sont conçues pour être alignées de sorte que la butée d'extrémité et le matériau biocompatible sont adjacents l'un à l'autre de manière serrée.

5. Clou orthopédique (10) selon l'une quelconque des revendications précédentes, **caractérisé en ce que** la partie conique comprend un filetage (26), et **en ce que** le matériau biocompatible est agencé par-dessus le filetage.

6. Clou orthopédique (10) selon la revendication 5, **caractérisé en ce que** le filetage comprend des crêtes de filetage arrondies et un fond de filetage arrondi.

7. Clou orthopédique (10) selon l'une quelconque des revendications 5 et 6, **caractérisé en ce que** le filetage (26) est traversé par au moins une rainure s'étendant de manière axiale.

8. Clou orthopédique (10) selon la revendication 7, **caractérisé en ce que** trois de ces rainures agencées à un angle de 120° l'une par rapport à l'autre sont fournies, le filetage étant traversé par chacune des trois rainures.

9. Clou orthopédique (10) selon l'une quelconque des revendications précédentes, **caractérisé par** une incrustation de métal (40) gainée dans la région distale du corps en plastique (30) et comprenant une ouverture destinée à recevoir une vis de blocage.

10. Clou orthopédique (10) selon l'une quelconque des revendications précédentes, **caractérisé en ce que** le matériau biocompatible est un polymère.

11. Clou orthopédique (10) selon la revendication 10, **caractérisé en ce que** le polymère est choisi dans le groupe de polymères constitué par le polyétheréthercétone (PEEK), le polyétheréthercétone renforcé avec de la fibre de carbone et le polyamide (PA).

12. Procédé destiné à produire un implant (10) destiné à l'ostéosynthèse, comprenant les étapes de :
- fabrication d'un composant porteur (20) à partir de métal de sorte que le composant porteur comprend une extrémité proximale, une extrémité distale, une partie conique entre l'extrémité proximale et l'extrémité distale, et un axe longitudinal, et
- gainage du composant porteur dans un matériau biocompatible (30) de sorte que le matériau biocompatible est agencé sur la partie conique ;
dans lequel le métal est le titane,
et le composant porteur (20) comprend une rainure (22) faite de manière transversale par rapport à l'axe longitudinal de celui-ci, dans laquelle rainure le matériau biocompatible entre en prise.

13. Procédé selon la revendication 12, **caractérisé en ce que** le gainage a lieu au moyen d'un surmoulage.

14. Procédé selon l'une quelconque des revendications précédentes 12 et 13, **caractérisé en ce que** l'implant est un clou orthopédique.

15. Procédé destiné à produire un implant destiné à l'ostéosynthèse, comprenant les étapes de :
fabrication d'un composant porteur (20) à partir de titane de sorte que le composant porteur comprend ce qui suit :
une extrémité proximale comprenant une butée d'extrémité (24) qui s'étend de manière radiale à partir du composant porteur et comprend une surface latérale ;
une extrémité distale ;
une partie conique agencée entre l'extrémité proximale et l'extrémité distale et comprenant un filetage ayant des crêtes de filetage arrondies et un fond de filetage arrondi, dans lequel le filetage (26) est traversé par au moins une rainure s'étendant de manière axiale, et
un axe longitudinal ; et
gainage d'une partie du composant porteur dans un matériau biocompatible (30) de sorte que le matériau biocompatible est agencé par-dessus le filetage, dans lequel la surface latérale de la butée d'extrémité et une surface du matériau biocompatible sont conçues pour être alignées de sorte que la butée d'extrémité et le matériau biocompatible sont adjacents l'un à l'autre de manière serrée.

16. Procédé destiné à produire un implant destiné à l'ostéosynthèse, comprenant les étapes de :
fabrication d'un composant porteur (20) à partir de titane de sorte que le composant porteur comprend ce qui suit :
une extrémité proximale comprenant une butée d'extrémité (24) qui s'étend de manière radiale à partir du composant porteur et comprend une surface latérale ;
une extrémité distale ;
une partie conique agencée entre l'extrémité proximale et l'extrémité distale et comprenant un filetage ayant des crêtes de filetage arrondies et un fond de filetage arrondi, dans lequel le filetage (26) est traversé par trois rainures s'étendant axialement,
et un axe longitudinal ; et
surmoulage d'une partie du composant porteur avec un polymère biocompatible (30) de sorte que le polymère biocompatible est agencé par-dessus le filetage et les trois rainures s'étendant de manière axiale, dans lequel la surface latérale de la butée d'extrémité et une surface du polymère biocompatible sont conçues pour être alignées de sorte que la butée d'extrémité et le matériau biocompatible sont adjacents l'un à l'autre de manière serrée.
